(19) Europäisches Patentamt
European Patent Office
Office européen des brevets

(11) Veröffentlichungsnummer: **0 008 686**
**B1**

(12) # EUROPÄISCHE PATENTSCHRIFT

(45) Veröffentlichungstag der Patentschrift: **20.04.83**

(21) Anmeldenummer: **79102823.6**

(22) Anmeldetag: **06.08.79**

(51) Int. Cl.³: **C 07 D 211/14**, C 07 D 295/02, C 07 D 265/30

(54) **Verfahren zur Herstellung von Phenyl-propyl-morpholin- und -piperidin-Derivaten.**

(30) Priorität: **08.08.78 CH 8407/78**

(43) Veröffentlichungstag der Anmeldung:
**19.03.80 Patentblatt 80/6**

(45) Bekanntmachung des Hinweises auf die Patenterteilung:
**20.04.83 Patentblatt 83/16**

(84) Benannte Vertragsstaaten:
**AT BE CH DE FR GB IT NL**

(56) Entgegenhaltungen:
**EP - A - 0 000 333**
**DE - A - 2 752 135**
**US - A - 3 965 105**

**"Friedel-Crafts and Related Reactions" (Alkylation and Related Reactions) Vol. II, Part 1 G. A. OLAK Interscience Publishers (1964)**

**"Friedel-Crafts and Related Reactions (acylation and Related Reactions) Vol. III, Part 2 G. A. OLAK Interscience Publishers (1964)**

(73) Patentinhaber: **F. HOFFMANN-LA ROCHE & CO. Aktiengesellschaft**
**CH-4002 Basel (CH)**

(72) Erfinder: **Pfiffner, Albert, Dr.**
**Grampenweg 10**
**CH-8180 Bülach (CH)**

(74) Vertreter: **Lederer, Franz, Dr. et al,**
**Patentanwälte Lederer, Meyer Lucile-Grahn-Strasse 22**
**D-8000 München 80 (DE)**

(56) References cited:
**Doklady Akademii Nauk SS.S.R. Vol. 133, No. 4, pp 843—846, August 1960**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 686 B1

Verfahren zur Herstellung von Phenyl-propyl-morpholin-und-piperidin-Derivaten

Die Erfindung betrifft ein verbessertes Verfahren zur Herstellung von Verbindungen der allgemeinen Formel I

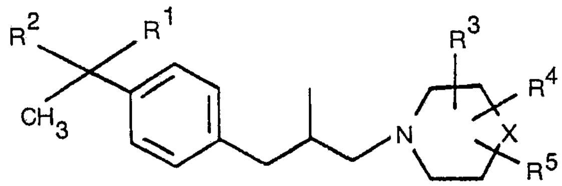

(I)

worin $R^1$ und $R^2$ Niederalkyl mit 1—4 Kohlenstoffatomen, Halo-niederalkyl mit 1—4 Kohlenstoffatomen, oder $R^1$ und $R^2$ zusammen mit dem Kohlenstoffatom an das sie gemeinsam gebunden sind, Cycloalkyl mit 3—7 Kohlenstoffatomen oder Niederalkyl substituiertes Cycloalkyl mit 4 bis 9 Kohlenstoffatomen bedeuten, $R^3$, $R^4$ und $R^5$ Wasserstoff oder Niederalkyl mit 1—4 Kohlenstoffatomen bedeuten,

X für eine Methylengruppe oder ein Sauerstoffatom steht; und Salzen von solchen Verbindungen, die basischen Charakter aufweisen, dadurch gekennzeichnet, dass man eine Verbindung der allgemeinen Formel II

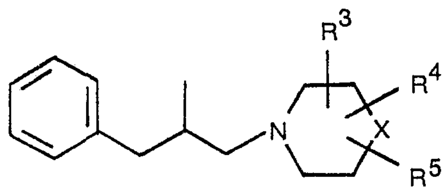

(II)

worin $R^3$, $R^4$, $R^5$ und X die in der allgemeinen Formel I angegebene Bedeutung besitzen, in Anwesenheit eines chlorierten Kohlenwasserstoffs mit einer Verbindung umsetzt, die unter den Bedingungen der Friedel-Crafts-Reaktion ein Carboniumion der allgemeinen Formel III

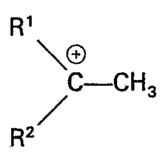

(III)

worin $R^1$ und $R^2$ die in der allgemeinen Formel I angegebene Bedeutung besitzen, liefert.

Alkylreste mit 1 bis 4 Kohlenstoffatomen sind geradkettige oder verzweigte Kohlenwasserstoffreste wie Methyl, Aethyl, Propyl, Isopropyl, Butyl, Isobutyl und tert. Butyl.

Der Ausdruck Halo-niederalkyl umfasst eine nieder molekulare Alkylgruppe in der eines der Wasserstoffatome durch ein Halogen atom, verzugsweise Fluor, Chlor oder Brom ersetzt ist. Die Bezeichnung "Cycloalkyl" umschreibt einen alicyclischen Kohlenwasserstoffrest mit 3 bis 7 Kohlenstoffatomen wie Cyclopropyl, Cyclobutyl, Cyclopentyl, Cyclohexyl oder Cycloheptyl.

Als Salze für die Verbindungen der allgemeinen Formel I, die basischen Charakter aufweisen, kommen Salze mit physiologisch verträglichen Säuren in Frage. Hierzu gehören vorzugsweise die Halogenwasserstoffsäuren, wie z.B. die Chlorwasserstoffsäure und die Bromwasserstoffsäure, ferner Phosphorsäure, Salpetersäure, ausserdem mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure und Milchsäure, und schliesslich Sulfonsäuren, wie die 1,5-Naphthalindisulfonsäure. Die Herstellung von derartigen Salzen erfolgt in an sich bekannter Weise.

Die Verbindungen der allgemeinen Formel I besitzen fungicide Wirkung und eignen sich besonders zur Bekämpfung von Fungi in der Landwirtschaft und im Gartenbau.

Bevorzugte Verbindungen der allgemeinen Formel I sind:

1-[3-(p-tert.Butylphenyl)-2-methyl-propyl]-piperidin;
1-[3-(p-tert.Butyl-phenyl)-2-methyl-propyl]-3-methyl-piperidin;
1-[3-(p-tert.Butyl-phenyl)-2-methyl-propyl]-3,5-dimethyl-piperidin;
4-[3-(p-tert.Butyl-phenyl)-2-methyl-propyl]-2,6-dimethyl-morpholin;
1-/3-[p-(2-Chlor-1,1-dimethyl-äthyl)-phenyl]-2-methyl-propyl/-piperdin;
1-/3-[p-(2-Chlor-1,1-dimethyl-äthyl)-phenyl]-2-methyl-propyl/-3,5-dimethyl-piperidin;
4-/3-[p-(2-Chlor-1,1-dimethyl-äthyl)-phenyl]-2-methyl-propyl/-2,6-dimethyl-morpholin;
1-[3-(p-tert.Amyl-phenyl)-2-methyl-propyl]-piperidin;

1-[3-(p-tert.Amyl-phenyl)-2-methyl-propyl]-3-methyl-piperidin;
1-[3-(p-tert.Amyl-phenyl)-2-methyl-propyl]-3,5-dimethyl-piperidin;
4-[3-(p-tert.Amyl-phenyl)-2-methyl-propyl]-2,6-dimethyl-morpholin;
1-/3-[p-(1-Cyclohexyl-1-methyl)-phenyl]-2-methyl-propyl/-piperidin;
1-/3-[p-(1-Cyclohexyl-1-methyl)-phenyl]-2-methyl-propyl/-3,5-dimethyl-piperidin;
4-/3-[p-(1-Cyclohexyl-1-methyl)-phenyl]-2-methyl-propyl/-2,6-dimethyl-morpholin.

Das erfindungsgemässe Verfahren ermöglicht bei Verwendung von billigeren Ausgangsmaterialien die Herstellung der Verbindungen der allgemeinen Formel I rationeller, in weniger Stufen und mit besserer Ausbeute als z.B. mit den Verfahren, die in den Deutschen Offenlegungsschriften Nos. 2656747, 2752096 und 2752135 beschrieben sind.

Die Alkylierung der Verbindung der allgemeinen Formel II erfolgt in Anwesenheit einer geeigneten Menge eines Friedel-Crafts Katalysators. Als Katalysator eignen sich die bekannten Friedel-Crafts Katalysatoren wie z.B. Aluminiumchlorid, Eisenchloride, Zinkchloride, Bortrifluorid, Zinnchlorid, Fluorwasserstoff, Schwefelsäure und Phosphorsäure. Für die Zwecke der Erfindung ist Schwefelsäure besonders bevorzugt.

Alschlorierte Kohlenwasserstoffe, in deren Gegenwart die Reaktion durchgeführt wird, eignen sich besonders Chloroform, Aethylendichlorid und Methylenchlorid, wobei Methylenchlorid besonders bevorzugt ist. Die Temperatur der Alkylierungsreaktion ist nicht kritisch, liegt jedoch in der Regel zwischen 0—50°C, vorzugsweise zwischen 18—20°C.

Vorzugsweise und insbesondere im Falle der Verwendung von Schwefelsäure wird nach Beendigung der Reaktion das erhaltene Produkt in der Salzform mit einem inerten organischen Lösungsmittel wie z.B. Methylenchlorid extrahiert. Falls erwünscht kann das freie Amin mit einer geeigneten Base wie Natronlauge, Kalilauge, Soda, Potasche oder Calciumhydroxid erhalten werden.

Bevorzugte Verbindungen welche die Carboniumionen der allgemeinen Formel III liefern sind
1-(2-Methyl-3-phenyl-propyl)-piperidin;
1-(2-Methyl-3-phenyl-propyl)-3-methyl-piperidin;
1-(2-Methyl-3-phenyl-propyl)-3,5-dimethyl-piperidin;
4-(2-Methyl-3-phenyl-propyl)-2,6-dimethyl-morpholin.

Bevorzugte Verbindungen welche die Carboniumionen der allgemeinen Formel III lieferen sind Alkene wie Isobutylen, 3-Chlor-2-methyl-1-propen oder 1-Methylcyclohexen; oder Alkohole insbesondere tert. Alkohole wie tert. Butyl-alkohol oder 2-Methyl-2-butanol; oder sekundäre Alkohole wie 2-Methylcyclohexanol oder 4-Methylcyclohexanol.

Die Verbindungen der allgemeinen Formel II sind teilweise neu. Sie können hergestellt werden indem man eine Verbindung der allgemeinen Formel IV

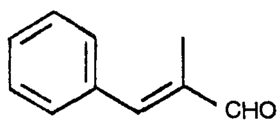

(IV)

mit einer Verbindung der allgemeinen Formel V

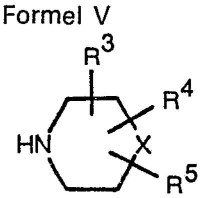

(V)

worin $R^3$, $R^4$ und $R^5$ Wasserstoff oder Niederalkyl mit 1—4 Kohlenstoffatomen bedeuten, X für eine Methylengruppe oder ein Sauerstoffatom steht, vermischt und katalytisch hydriert.

Verbindungen der allgemeinen Formel V können, wenn mindestens zwei der Substituenten $R^3$, $R^4$ und $R^5$ Niederalkyl bedeuten und nicht am selben Kohlenstoffatom gebunden sind, erwünschtenfalls als reine Isomere eingesetzt werden.

Als Lösungsmittel wird vorzugsweise ein organisches Lösungsmittel z.B. ein Alkohol, wie Methanol verwendet. Die Temperatur ist nicht kritisch, liegt jedoch in der Regel zwischen 0°—50°C.

Bei der katalytischen Hydrierung dienen die üblichen Hydrierungskatalysatoren wie z.B. Platin, Raney Nickel oder Palladium, wobei 5% Palladiumkohle besonders bevorzugt wird.

Die nachstehenden Beispiele illustrieren die Erfindung.

## Beispiel 1

800 g 1-(2-Methyl-3-phenyl-propyl)-piperidin in 3000 ml Methylenchlorid werden vorgelegt und innerhalb 1,5 Stunden, unter Sole-Kühlung und intensivem Rühren bei 5—10°C Innentemperatur, 2040 g 95—96%-ige Schwefelsäure zugetropft. Anschliessend wird bei 15—20°C Innentemperatur während 2 Stunden 266 g Isobutylen eingeleitet und unter Eiskühlung 1620 ml Wasser zugetropft. Die rot gefärbte Schwefelsäurelösung entfärbt sich und das Produkt löst sich als Hydrosulfat in der Methylenchloridphase. Die farblose Schwefelsäurelösung (untere Phase) wird abgetrennt und nochmals mit Methylenchlorid nachextrahiert. Die vereinigten Methylenchloridextrakte werden mit Wasser gewaschen und das Produkt durch portionenweise Zugabe von 1060 g krist. Natriumcarbonat unter Rühren in der Methylenchloridphase freigesetzt. Die Methylenchloridphase wird von der entstanden wässrigen Phase abgetrennt, mit Wasser nachgewaschen und das Methylenchlorid abgedampft. Durch Destillation an einer 30 cm-Goodloe-Silberkolonne wird reines 1-[3-(p-tert.Butylphenyl)-2-methyl-propyl]-piperidin erhalten; Sdp. 118°C/0,07 Torr.

In analoger Weise erhält man aus:

— 1-(2-Methyl-3-phenyl-propyl)-3-methyl-piperidin und Isobutylen das 1-[3-(p-tert.Butyl-phenyl)-2-methyl-propyl]-3-methyl-piperidin, Sdp. 116°C/0,02 Torr.,

— 1-(2-Methyl-3-phenyl-propyl)-3,5-dimethyl-piperidin und Isobutylen das 1-[3-(p-tert.Butyl-phenyl)-2-methyl-propyl]-3,5-dimethyl-piperidin, Sdp. 128—130°C/0,001 Torr.,

— 4-(2-Methyl-3-phenyl-propyl)-2,6-dimethyl-morpholin und Isobutylen das 4-[3-(p-tert.Butyl-phenyl]-2-methyl-propyl]-2,6-dimethyl-morpholin, Sdp. 134—136°C/0,03 Torr.,

— 1-(2-Methyl-3-phenyl-propyl)-piperidin und 3-Chlor-2-methyl-1-propen das 1-/3/[p-(2-Chlor-1,1-dimethyläthyl)-phenyl]-2-methyl-propyl/-piperidin, Sdp. 139—140°C/0,04 Torr.,

— 1-(2-Methyl-3-phenyl-propyl)-3,5-dimethyl-piperidin und 3-Chloro-2-methyl-1-propen das 1-/3-[p-(2-Chlor-1,1-dimethyl-äthyl)-phenyl]-2-methyl-propyl/-3,5-dimethyl-piperidin, Sdp. 155—156°C/ 0,04 Torr.,

/3-[p-(2-Chlor-1,1-dimethyl-äthyl)-phenyl]-2-methyl-propyl/-2,6-dimethyl-morpholin, Sdp. 155°C/0,04 Torr.

155°C/0,04 Torr.

Beispiel 2

100 g 1-(2-Methyl-3-phenyl-propyl)-piperidin in 375 ml Methylenchlorid werden vorgelegt und innerhalb 1,5 Stunden, unter Sole-Kühlung und intensivem Rühren bei 10°C Innentemperatur, 255 g 95—96%-ige Schwefelsäure zugetropft. Anschliessend wird bei 20°C 42,5 g tert. Butylalkohol zugetropft und man lässt 2 Stunden bei Raumtemperatur nachreagieren. Die Aufarbeitung erfolgt in Analogie zu Beispiel 1. Das Rohprodukt wird durch fraktionierte Destillation an einer Goodloe-Silberkolonne gereinigt. Es wird reines 1-[3-(p-tert.Butyl-phenyl)-2-methyl-propyl]-piperidin erhalten; Sdp. 118°C/0,07 Torr.

In analoger Weise erhält man aus:

— 1-(2-Methyl-3-phenyl-propyl)-3-methyl-piperidin und tert. Butylalkohol das 1-[3-(p-tert.Butyl-phenyl)-2-methyl-propyl]-3-methyl-piperidin, Sdp. 116°C/0,02 Torr.,

— 1-(2-Methyl-3-phenyl-propyl)-3,5-dimethyl-piperidin und tert. Butylalkohol das 1-[3-(p-tert.Butyl-phenyl)-2-methyl-propyl]-3,5-dimethyl-piperidin, Sdp. 128—130°C/0,001 Torr.,

— 4-(2-Methyl-3-phenyl-propyl)-2,6-dimethyl-morpholin und tert. Butylalkohol das 4-[3-(p-tert.Butyl-phenyl)-2-methyl-propyl]-2,6-dimethyl-morpholin, Sdp. 134—136°C/0,03 Torr.,

— 1-(2-Methyl-3-phenyl-propyl)-piperidin und 2-Methyl-2-butanol das 1-[3-(p-tert.Amyl-phenyl)-2-methyl-propyl]-piperidin, Sdp. 160°C/0,15 Torr.,

— 1-(2-Methyl-3-phenyl-propyl)-3-methyl-piperidin und 2-Methyl-2-butanol das 1-[3-(p-tert.Amyl-phenyl)-2-methyl-propyl]-3-methyl-piperidin, Sdp. 164°C/0,15 Torr.,

— 1-(2-Methyl-3-phenyl-propyl)-3,5-dimethyl-piperidin und 2-Methyl-2-butanol das 1-[3-(p-tert.Amyl-phenyl)-2-methyl-propyl]-3,5-dimethyl-piperidin, Sdp. 135°C/0,05 Torr.,

— 4-(2-Methyl-3-phenyl-propyl)-2,6-dimethyl-morpholin und 2-Methyl-2-butanol das 4-[3-(p-tert.Amyl-phenyl)-2-methyl-propyl]-2,6-dimethyl-morpholin, Sdp. 136—138°C/0,04 Torr.,

— 1-(2-Methyl-3-phenyl-propyl)-piperidin und 2-Methylcyclohexanol das 1-/3-[p-(1-Cyclohexyl-1-methyl)-phenyl]-2-methyl-propyl/-piperidin, Sdp. 140°C/0,04 Torr.,

— 1-(2-Methyl-3-phenyl-propyl)-3,5-dimethyl-piperidin und 2-Methyl-cyclohexanol das 1-/3-[p-(1-Cyclohexyl-1-methyl)-phenyl]-2-methyl-propyl/-3,5-dimethyl-piperidin, Sdp. 130—132°C/0,04 Torr.,

— 4-(2-Methyl-3-phenyl-propyl)-2,6-dimethyl-morpholin und 2-Methyl-cyclohexanol das 4-/3-[p-(1-Cyclohexyl-1-methyl)-phenyl]-2-methyl-propyl/-2,6-dimethyl-morpholin, Sdp. 160—162°C/0,07 Torr.

Beispiel 3

1000 g $\alpha$-Methyl-zimtaldehyd, 10 l Methanol und 640 g Piperidin werden unter Stickstoffbegasung vorgelegt und 50 g Palladiumkohle zugegeben. Anschliessend wird unter Wasserkühlung bei 30°C Innentemperatur bis zur Beendidung der Wasserstoffaufnahme hydriert, von Katalysator abfiltriert und das Methanol am Vakuum abdestilliert. Das Rohprodukt wird destilliert. Es wird reines 1-(2-Methyl-3-phenyl-propyl)-piperidin erhalten, Sdp. 92°C/0,04 Torr.

In analoger Weise erhält man aus:

— $\alpha$-Methyl-zimtaldehyd und 3-Methyl-piperidin das 1-(2-Methyl-3-phenyl-propyl)-3-methyl-piperidin, Sdp. 103°C/0,04 Torr.,

— $\alpha$-Methyl-zimtaldehyd und 3,5-Dimethyl-piperidin das 1-(2-Methyl-3-phenyl-propyl)-3,5-dimethyl-piperidin, Sdp. 94°C/0,03 Torr.,

— $\alpha$-Methyl-zimtaldehyd und 2,6-Dimethyl-morpholin das 4-(2-Methyl-3-phenyl-propyl)-2,6-dimethyl-morpholin, Sdp. 92°C/0,03 Torr.

**Patentansprüche**

1. Verfahren zur Herstellung von Verbindungen der allgemeinen Formel I

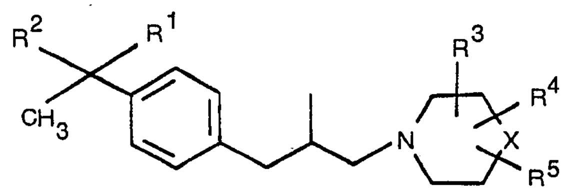

(I)

worin $R^1$ und $R^2$ Niederalkyl mit 1—4 Kohlenstoffatomen, Halo-niederalkyl mit 1—4 Kohlenstoffatomen, oder $R^1$ und $R^2$ zusammen mit dem Kohlenstoffatom an das sie gemeinsam gebunden sind, Cycloalkyl mit 3—7 Kohlenstoffatomen oder Niederalkyl substituiertes Cycloalkyl mit 4 bis 9 Kohlenstoffatomen bedeuten, $R^3$, $R^4$ und $R^5$ Wasserstoff oder Niederalkyl mit 1—4 Kohlenstoffatomen bedeuten,
X für eine Methylengruppe oder ein Sauerstoffatom steht; und Salzen von solchen Verbindungen die basischen Charakter aufweisen, dadurch gekennzeichnet, dass man eine Verbindung der allgemeinen Formel II

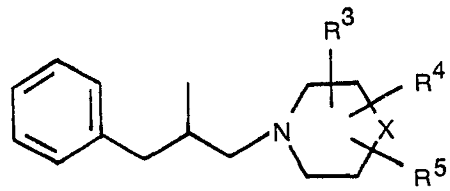

(II)

worin $R^3$, $R^4$, $R^5$ und X die in Formel I angegebene Bedeutung besitzen,
in Anwesenheit eines chlorierten Kohlenwasserstoffes mit einer Verbindung umsetzt, die unter den Bedingungen der Friedel-Crafts-Reaktion ein Carboniumion der allgemeinen Formel III

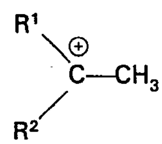

(III)

worin $R^1$ und $R^2$ die in der allgemeinen Formel I angegebene Bedeutung besitzen, der allgemeinen liefert.

2. Verfahren nach Anspruch 1, dadurch gekennzeichnet, dass man die Reaktion in Gegenwart von Schwefelsäure durchführt.

3. Verfahren nach einem der Ansprüche 1 und 2, dadurch gekennzeichnet, dass nach Beendigung der Reaktion das erhaltene Produkt in Salzform mit einem inerten organischen Losssungsmittel extrahiert wird.

4. Verfahren nach einem der Ansprüche 1 bis 3, dadurch gekennzeichnet, dass die Verbindung, welche das Carboniumion der allgemeinen Formel III liefert Isobutylen, 3-Chlor-2-methyl-1-propen oder 1-Methylcyclohexen ist.

5. Verfahren nach Anspruch 4, dadurch gekennzeichnet, dass diese Verbindung Isobutylen ist.

6. Verfahren nach Anspruch 4, dadurch gekennzeichnet, dass diese Verbindung 3-Chlor-2-methyl-1-propen ist.

7. Verfahren nach einem der Ansprüche 1 bis 3, dadurch gekennzeichnet, dass die Verbindung, welche das Carboniumion der allgemeinen Formel III liefert tert. Butylalkohol, 2-Methyl-2-butanol, 2-Methylcyclohexanol oder 4-Methylcyclohexanol ist.

8. Verfahren nach Anspruch 7, dadurch gekennzeichnet, dass diese Verbindung tert. Butylalkohol ist.

9. Verfahren nach Anspruch 7, dadurch gekennzeichnet, dass diese Verbindung 2-Methyl-2-butanol ist.

10. Verfahren nach Anspruch 7, dadurch gekennzeichnet, dass diese Verbindung 2-Methylcyclohexanol ist.

11. Verfahren nach einem der Ansprüche 1 bis 10, dadurch gekennzeichnet, dass die Verbindung der allgemeinen Formel II 1-(2-Methyl-3-phenyl-propyl)-piperidin ist.

12. Verfahren nach einem der Ansprüche 1 bis 10, dadurch gekennzeichnet, dass die Verbindung

der allgemeinen Formel II 1-(2-Methyl-3-phenyl-propyl)-3-methyl-piperidin ist.

13. Verfahren nach einem der Ansprüche 1 bis 10, dadurch gekennzeichnet, dass die Verbindung der allgemeinen Formel II 1-(2-Methyl-3-phenyl-propyl)-3,5-dimethyl-piperidin ist.

14. Verfahren nach einem der Ansprüche 1 bis 10, dadurch gekennzeichnet, dass die Verbindung der allgemeinen Formel II 4-(2-Methyl-3-phenyl-propyl)-2,6-dimethyl-morpholin ist.

15. Verfahren nach einem der Ansprüche 1 bis 5 und 11 zur Herstellung von 1-[3-(p-tert.Butyl-phenyl)-2-methyl-propyl]-piperidin, dadurch gekennzeichnet, dass man 1-(2-Methyl-3-phenyl-propyl)-piperidin mit Isobutylen umsetzt.

16. Verfahren nach einem der Ansprüche 1 bis 3, 7, 8 und 11 zur Herstellung von 1-[3-(p-tert.Butyl-phenyl)-2-methyl-propyl]-piperidin, dadurch gekennzeichnet, dass man 1-(2-Methyl-3-phenyl-propyl)-piperidin mit tert. Butylalkohol umsetzt.

17. Verfahren nach einem der Ansprüche 1 bis 5 und 14 zur Herstellung von 4-[3-(p-tert.Butyl-phenyl)-2-methyl-propyl]-2,6-dimethyl-morpholin, dadurch gekennzeichnet, dass man 4-(2-Methyl-3-phenyl-propyl)-2,6-dimethyl-morpholin mit Isobutylen umsetzt.

18. Verfahren nach einem der Ansprüche 1 bis 3, 7, 8 und 14 zur Herstellung von 4-[3-(p-tert.Butyl-phenyl)-2-methyl-propyl]-2,6-dimethyl-morpholin, dadurch gekennzeichnet, dass man 4-(2-Methyl-3-phenyl-propyl)-2,6-dimethyl-morpholin mit tert. Butylalkohol umsetzt.

## Claims

1. Process for the manufacture of compounds of general formula I

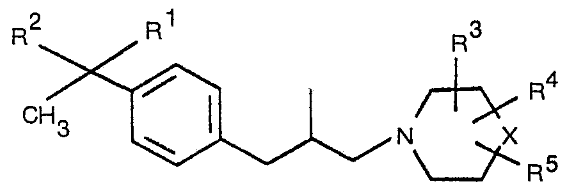

(I)

wherein $R^1$ and $R^2$ signify lower alkyl with 1—4 carbon atoms, halo-lower alkyl with 1—4 carbon atoms, or $R^1$ and $R^2$ together with the carbon atom to which they are commonly attached signify cycloalkyl with 3—7 carbon atoms or lower alkyl-substituted cycloalkyl with 4 to 9 carbon atoms, $R^3$, $R^4$ and $R^5$ signify hydrogen or lower alkyl with 1—4 carbon atoms,
X stands for a methylene group or an oxygen atom; and salts of such compounds which have basic character, characterized by reacting a compound of general formula II

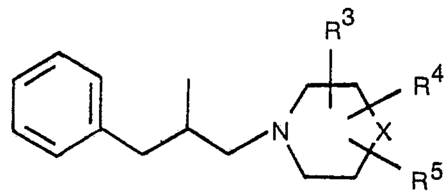

(II)

wherein $R^3$, $R^4$, $R^5$ and X have the significance given in formula I,
in the presence of a chlorinated hydrocarbon with a compound which under the conditions of the Friedel-Crafts reaction yields a carbonium ion of general formula III

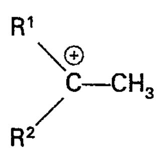

(III)

wherein $R^1$ and $R^2$ have the significance given in general formula I.

2. Process according to claim 1, characterized in that the reaction is carried out in the presence of sulphuric acid.

3. Process according to any one of claims 1 and 2, characterized in that after completion of the reaction the product obtained is extracted in salt form with an inert organic solvent.

4. Process according to any one of claims 1 to 3, characterized in that the compound which yields the carbonium ion of general formula III is isobutylene, 3-chloro-2-methyl-1-propene or 1-methylcyclohexene.

5. Process according to claim 4, characterized in that this compound is isobutylene.

6. Process according to claim 4, characterized in that this compound is 3-chloro-2-methyl-1-propene.

7. Process according to any one of claims 1 to 3, characterized in that the compound which yields the carbonium ion of general formula III is tert.butyl alcohol, 2-methyl-2-butanol, 2-methylcyclohexanol or 4-methylcyclohexanol.

8. Process according to claim 7, characterized in that this compound is tert.butyl alcohol.

9. Process according to claim 7, characterized in that this compound is 2-methyl-2-butanol.

10. Process according to claim 7, characterized in that this compound is 2-methylcyclohexanol.

11. Process according to any one of claims 1 to 10, characterized in that the compound of general formula II is 1-(2-methyl-3-phenyl-propyl)-piperidine.

12. Process according to any one of claims 1 to 10, characterized in that the compound of general formula II is 1-(2-methyl-3-phenyl-propyl)-3,5-dimethyl-piperidine.

13. Process according to any one of claims 1 to 10, characterized in that the compound of general formula II is 1-(2-methyl-3-phenyl-propyl)-3,5-dimethyl-piperidine.

14. Process according to any one of claims 1 to 10, characterized in that the compound of general formula II is 4-(2-methyl-3-phenyl-propyl)-2,6-dimethyl-morpholine.

15. Process according to any one of claims 1 to 5 and 11 for the manufacture of 1-[3-(p-tert.butyl-phenyl)-2-methyl-propyl]-piperidine, characterized by reacting 1-(2-methyl-3-phenyl-propyl)-piperidine with isobutylene.

16. Process according to any one of claims 1 to 3, 7, 8 and 11 for the manufacture of 1-[3-(p-tert.butyl-phenyl)-2-methyl-propyl]-piperidine, characterized by reacting 1-(2-methyl-3-phenyl-propyl)-piperidine with tert.butyl alcohol.

17. Process according to any one of claims 1 to 5 and 14 for the manufacture of 4-[3-(p-tert.butyl-phenyl)-2-methyl-propyl]-2,6-dimethyl-morpholine, characterized by reacting 4-(2-methyl-3-phenyl-propyl)-2,6-dimethyl-morpholine with isobutylene.

18. Process according to any one of claims 1 to 3, 7, 8 and 14 for the manufacture of 4-[3-(p-tert.butyl-phenyl)-2-methyl-propyl]-2,6-dimethyl-morpholine, characterized by reacting 4-(2-methyl-3-phenyl-propyl)-2,6-dimethyl-morpholine with tert.butyl alcohol.

**Revendications**

1. Procédé de préparation de composés de formule générale I

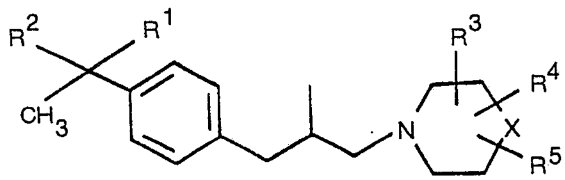

(I)

dans laquelle $R^1$ et $R^2$ représentent des groupes alkyles inférieurs en C1—C4, halogénoalkyles inférieurs en C1—C4, ou bien $R^1$ et $R^2$ forment ensemble et avec l'atome de carbone sur lequel ils sont fixés un groupe cycloalkyle en C3—C7 ou cycloalkyle en C4—C9 substitué par un groupe alkyle inférieur, $R^3$, $R^4$ et $R^5$ représentent l'hydrogène ou des groupes alkyles inférieurs en C1—C4, X représente un groupe méthylène ou un atome d'oxygène; et des sels de ces composés à caractère basique, caractérisé en ce que l'on fait réagir un composé de formule générale II

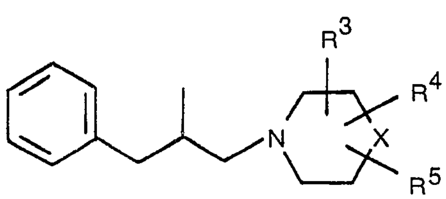

(II)

dans laquelle $R^3$, $R^4$, $R^5$ et X ont les significations indiquées pour la formule générale I, en présence d'un hydrocarbure chloré, avec un composé qui, dans les conditions de la réaction de Friedel-Crafts, fournit un ion carbonium de formule générale III

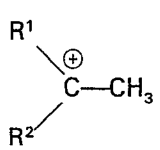

(III)

dans laquelle $R^1$ et $R^2$ ont les significations indiquées en référence à la formule générale I.

2. Procédé selon la revendication 1, caractérisé en ce que l'on effectue la réaction en présence d'acide sulfurique.

3. Procédé selon l'une des revendications 1 et 2, caractérisé en ce que, lorsque la réaction est terminée, on extrait le produit obtenut à l'état de sel à l'aide d'un solvant organique inerte.

4. Procédé selon l'une des revendications 1 à 3, caractérisé en ce que le composé qui fournit l'ion carbonium de formule générale III est l'isobutylène, le 3-chloro-2-méthyl-1-propène ou le 1-méthylcyclohexène.

5. Procédé selon la revendication 4, caractérisé en ce que ce composé est l'isobutylène.

6. Procédé selon la revendication 4, caractérisé en ce que ce composé est le 3-chloro-2-méthyl-1-propène.

7. Procédé selon l'une des revendications 1 à 3, caractérisé en ce que le composé qui fournit l'ion carbonium de formule générale III est l'alcool tert-butylique, le 2-méthyl-2-butanol, le 2-méthylcyclohexanol ou le 4-méthylcyclohexanol.

8. Procédé selon la revendication 7, caractérisé en ce que composé est l'aclool tert-butylique.

9. Procédé selon la revendication 7, caractérisé en ce que ce composé est le 2-méthyl-2-butanol.

10. Procédé selon la revendication 7, caractérisé en ce que ce composé est le 2-méthylcyclohexanol.

11. Procédé selon l'une des revendications 1 à 10, caractérisé en ce que le composé de formule générale II est la 1-(2-méthyl-3-phényl-propyl)-pipéridine.

12. Procédé selon l'une des revendications 1 à 10, caractérisé en ce que le composé de formule générale II est la 1-(2-méthyl-3-phényl-propyl)-3-méthyl-pipéridine.

13. Procédé selon l'une des revendications 1 à 10, caractérisé en ce que le composé de formule générale II est la 1-(2-méthyl-3-phényl-propyl)-3,5-diméthyl-pipéridine.

14. Procédé selon l'une des revendications 1 à 10, caractérisé en ce que le composé de formule générale II est la 4-(2-méthyl-3-phényl-propyl)-2,6-diméthyl-morpholine.

15. Procédé selon l'une des revendications 1 à 5 et 11, pour la préparation de la 1-[3-(p-tert-butyl-phényl)-2-méthyl-propyl]-pipéridine, caractérisé en ce que l'on fait réagir la 1-(2-méthyl-3-phényl-propyl)-pipéridine avec l'isobutylène.

16. Procédé selon l'une des revendications 1 à 3, 7, 8 et 11 pour la préparation de la 1-[3-(p-tert-butyl-phényl)-2-méthyl-propyl]-pipéridine, caractérisé en ce que l'on fait réagir la 1-(2-méthyl-3-phényl-propyl)-pipéridine avec l'alcool tert-butylique.

17. Procédé selon l'une des revendications 1 à 5 et 14 pour la préparation de la 4-[3-(p-tert-butyl-phényl)-2-méthyl-propyl]-2,6-diméthyl-morpholine, caractérisé en ce que l'on fait réagir la 4-(2-méthyl-3-phényl-propyl)-2,6-diméthyl-morpholine avec l'isobutylène.

18. Procédé selon l'une des revendications 1 à 3, 7, 8 et 14 pour la préparation de la 4-[3-(p-tert-butyl-phényl)-2-méthyl-propyl]-2,6-diméthyl-morpholine, caractérisé en ce que l'on fait réagir la 4-(2-méthyl-3-phényl-propyl)-2,6-diméthyl-morpholine avec l'alcool tert-butylique.